# FASCICULE DE BREVET EUROPEEN

(11) **EP 0 591 376 B1**
(45) Date de publication et mention de la délivrance du brevet: **26.08.1998**
(21) Numéro de dépôt: 92913975.6
(22) Date de dépôt: 25.06.1992
(51) Int. Cl.: C12N 15/37, C12Q 1/68, C12Q 1/70

(54) **SEQUENCES ADN DU PAPILLOMAVIRUS HPV42: APPLICATION AU DIAGNOSTIC**
DNS-SEQUENZEN VON HPV42 PAPILLOMAVIRUS :VERWENDUNGSMÖGLICHKEIT FÜR DIAGNOSE
ADN SEQUENCES OF THE HUMAN PAPILLOMAVIRUS TYPE 42, AND DIAGNOSTIC APPLICATION THEREOF

(30) Priorité: 28.06.1991 FR 9108125
(43) Date de publication de la demande: 13.04.1994
(73) Titulaire: INSTITUT PASTEUR, 75724 Paris Cédex 15 (FR)
(72) Inventeur: PHILIPP, Wolfgang, D-6500 Mainz (DE); SAPP, Martin, D-6500 Mainz (DE); COLE, Stewart, F-92140 Clamart (FR); HONORE, Nadine, F-92700 Colombes (FR)
(74) Mandataire: Gutmann, Ernest
(86) Numéro de dépôt international: FR9200586
(87) Numéro de publication internationale: WO9300435

(56) Documents cités:
- VIROLOGY vol. 186, no. 1, Janvier 1992, pages 331 - 334; PHILIPP, W. ET AL.: 'Human papillomavirus type 42 : new sequences, conserved genome organization'

## Description

L'invention concerne des séquences d'ADN déterminées dérivées du génome du papillomavirus HPV42, y inclue la séquence correspondante à son génome entier, ainsi que des ADNs recombinants, notamment vecteurs contenant tout ou partie de celles de ces séquences d'ADN qui codent pour des protéines de structure de ces papillomavirus ou parties de ces protéines. L'invention concerne également les cultures cellulaires transformées avec les dits ADNs recombinants dans des conditions leur permettant, le cas échéant, d'exprimer les séquences correspondantes dérivées du génome de HPV42 sous la forme des protéines correspondantes. L'invention est enfin relative à des kits de diagnostic mettant en oeuvre certains des produits définis ci-dessus ou ceux dont la description suit et leurs applications nouvelles plus particulièrement à la discrimination entre lésions épithéliales génitales bénignes et lésions de type carcinome ou pré-carcinome induites par des papillomavirus.

La plupart des 60 différents types ou plus de papillomavirus humains identifiés jusqu'à ce jour sont tous des virus épithéliotropiques présumés responsables de l'induction d'une croissance anormale du tissu infecté (1,39). Selon leurs spécificités tissulaires et leurs prédominances respectives dans les tumeurs malignes ou bénignes, les types de papillomavirus humains (HPV) ont été classés dans différents groupes, par exemple groupes à faible risque (HPV 6,11) (2,3), à haut risque (HPV 16, 18, 31, 33, 39, 57) (1, 4-8), HPVs génitaux et HPVs associés à des épidermodyaplasies verruciformes (HPV 5, 8, 19, 25, 47) (1,9-11), etc. Dans le but de mieux comprendre leur relation mutuelle et, s'il en était besoin, révéler la nature de leurs spécificités tissulaires, il a aussi été proposé de procéder à des comparaisons de séquences. Mais les tentatives d'établir des corrélations entre certaines caractéristiques du virus et certaines caractéristiques spécifiques de leurs génomes respectifs ne se sont guère révélés fructueuses jusqu'à ce jour. La démonstration de la difficulté à établir de telles corrélations est particulièrement illustrée par le résultat des études de séquences qui ont été conduites sur le papillomavirus HPV42, lequel avait déjà été isolé à partir de papillomas vulvaires, grâce à l'utilisation d'un ADN du type HPV32 oral (12) en tant que sonde sous des conditions de faible stringence (13). Selon cette dernière publication HPV42 s'était avéré être présent dans 3,5 % des lésions génitales, qui pour la plupart d'entre elles, présentaient des caractéristiques histologiques de condylomas ou de papillomas plats.

L'invention repose aussi sur la découverte de séquences particulières présentes dans HPV42, séquences qui en font l'originalité et qui permettent des détections particulièrement fines de papillomavirus du type HPV42 et des confirmations (ou infirmations) de résultats de diagnostics in vitro effectués avec des sondes contenant des séquences d'ADN issues d'autres papillomavirus. Ces séquences ou des fragments de ces séquences sont aussi utilisables pour la constitution de sondes d'hybridation particulièrement sensibles, en particulier d'amorces permettant des analyses par la méthode dite de PCR.

Dans ce qui suit, il sera fait usage d'un certain nombre d'observations issues d'expressions anglaises. Elles sont conservées dans le texte, pour faciliter la lecture du texte par les spécialistes coutumiers de ces abréviations.

Elles sont rappelées ci-après et sont suivies des expressions anglaises correspondantes complètes et de leurs traductions, entre des signes de parenthèse, en langue française. :
- NCR : non coding region (région non codante) ;
- ORF : open reading frame (phase de lecture ouverte) ;
- PVF : papillomavirus-enhancer associated factor (site de liaison du facteur associé à l'amplificateur de papillomavirus) ;
- GRE : consensus glucocorticoid responsive element (élément consensus réagissant à un glucocorticoïde) ;
- bp : base pairs (paires de bases) ;
- PCR : polymarase chain reaction (amplification par polymérisation de chaînes) ;
- nt : nucléotide.

Avant de procéder plus avant à la description de ces séquences ou fragments de séquences, il est proposé de faire un bref retour sur l'état antérieur de la technique et, ensuite, de fournir une description détaillée du génome de HPV42.

La suite de l'exposé sera faite par référence aux figures ci-jointes, et dont les légendes suivent :

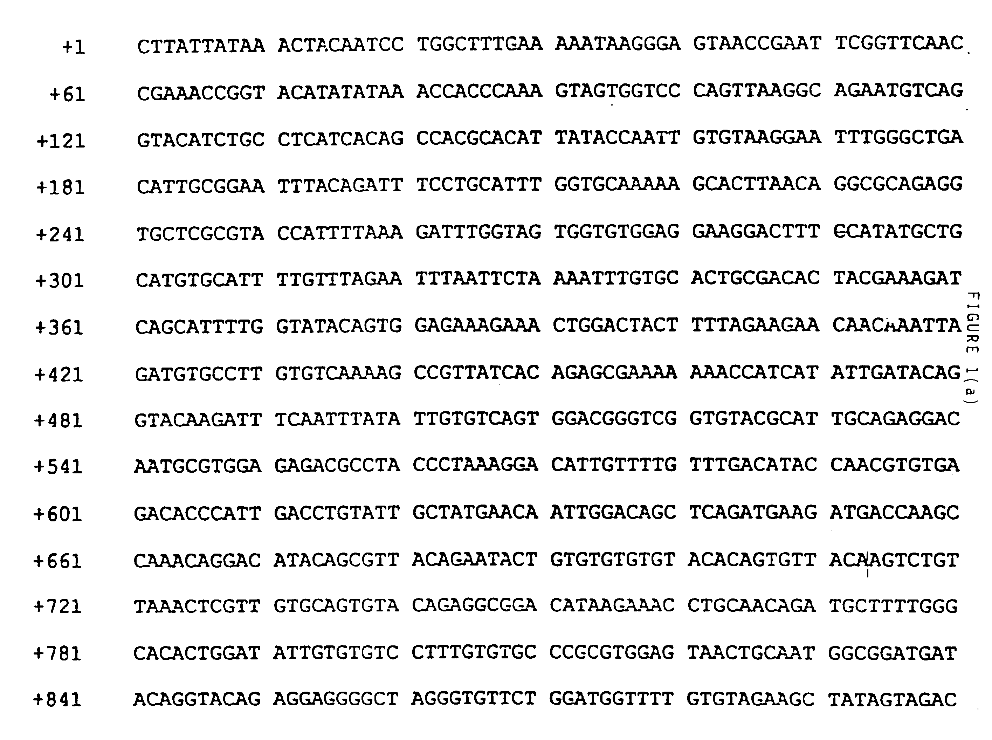

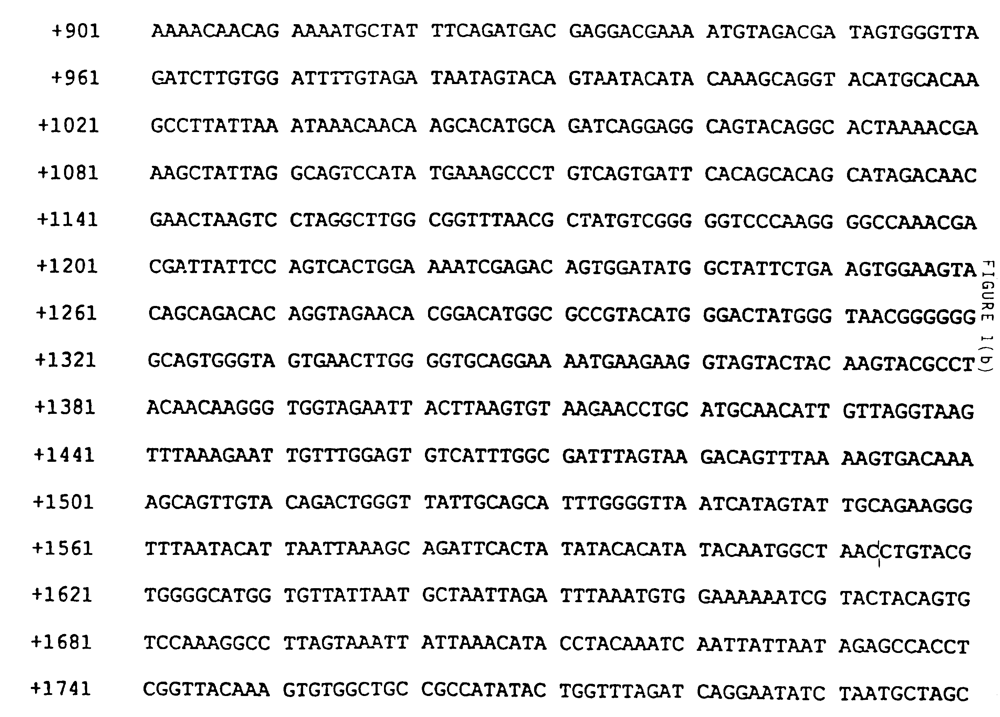

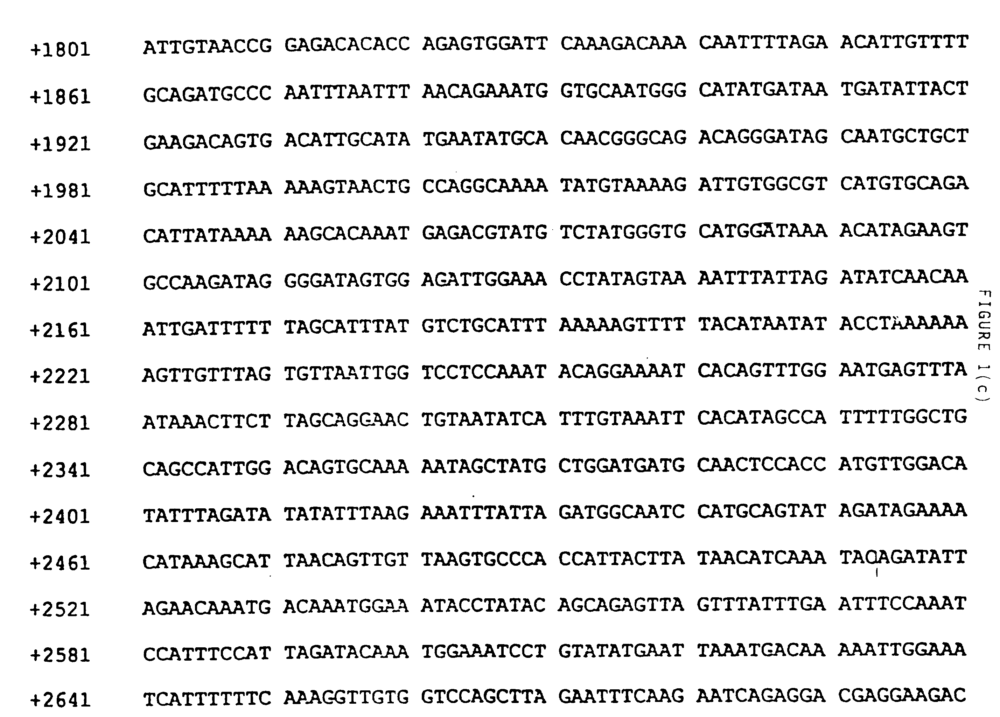

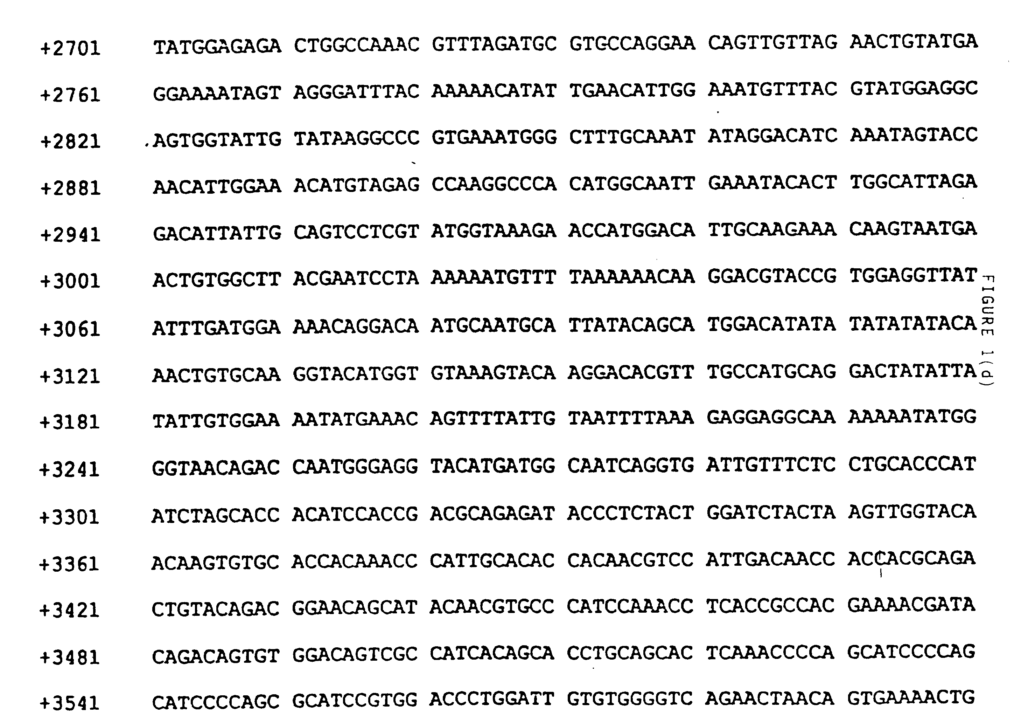

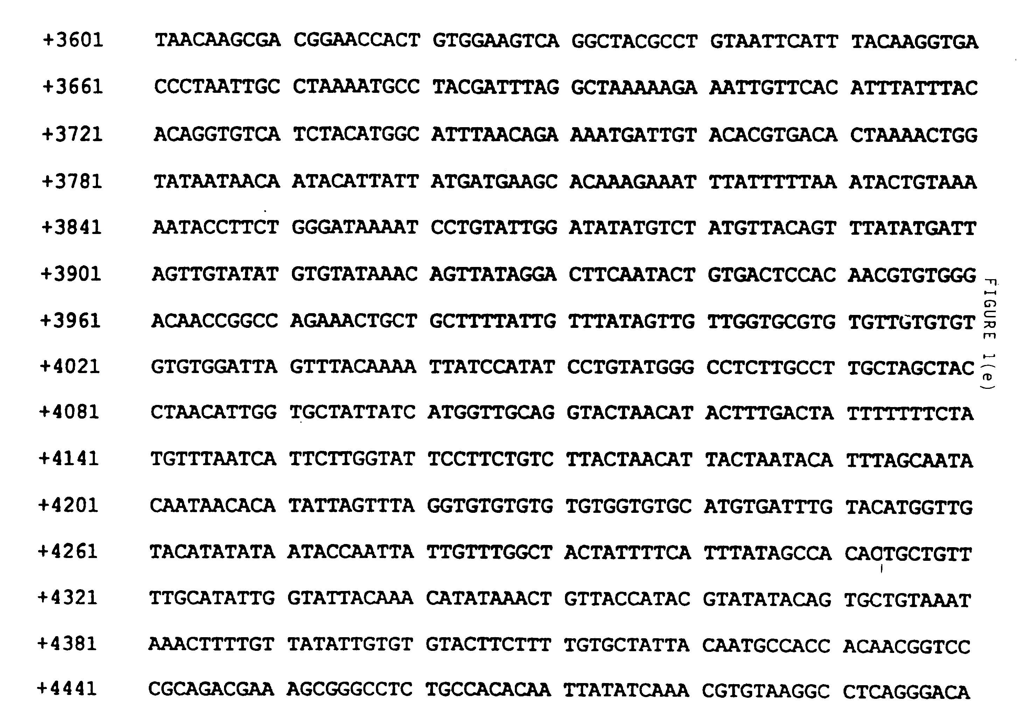

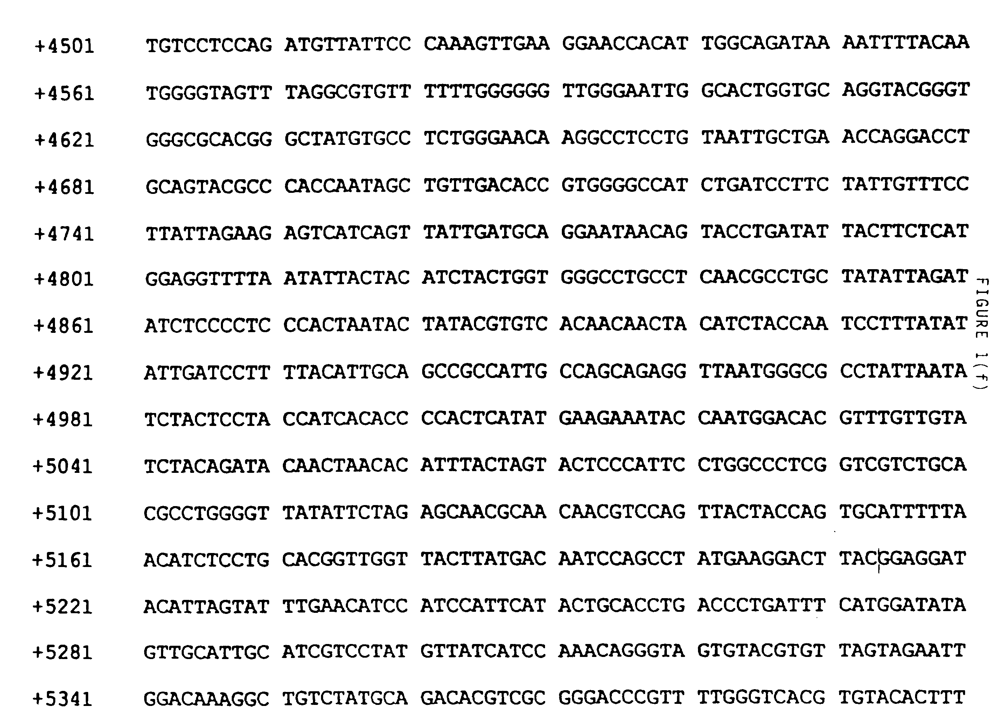

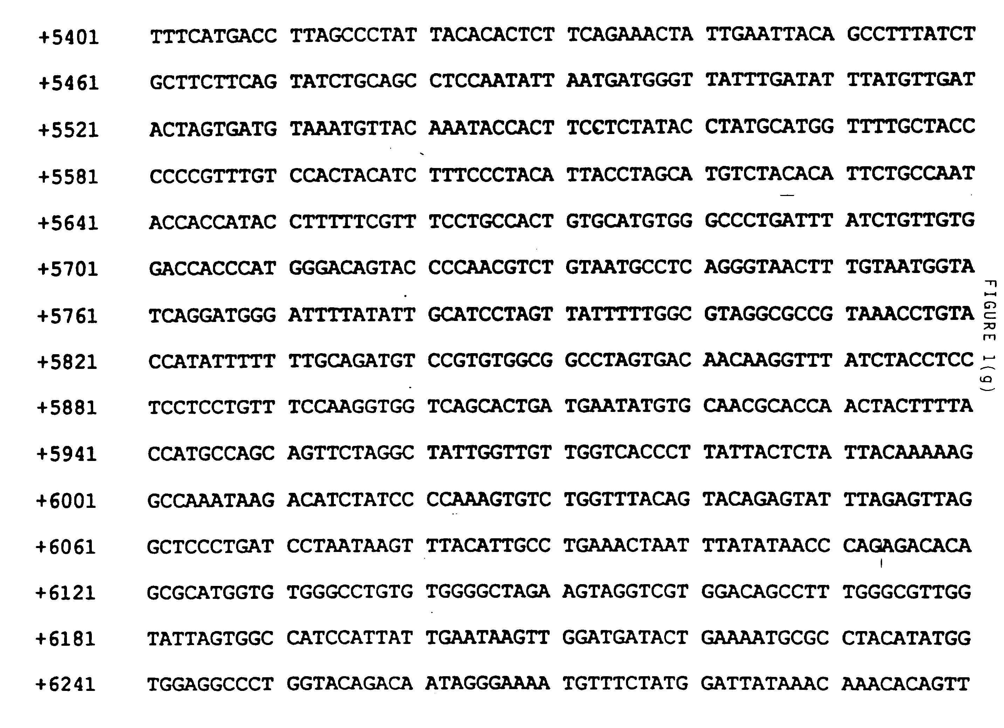

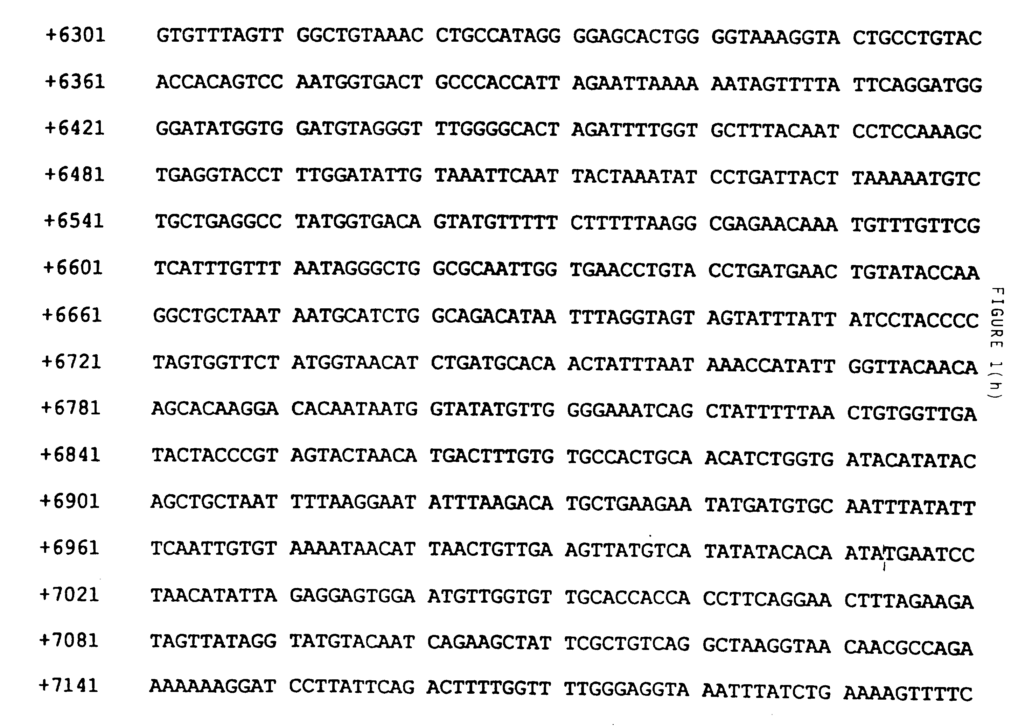

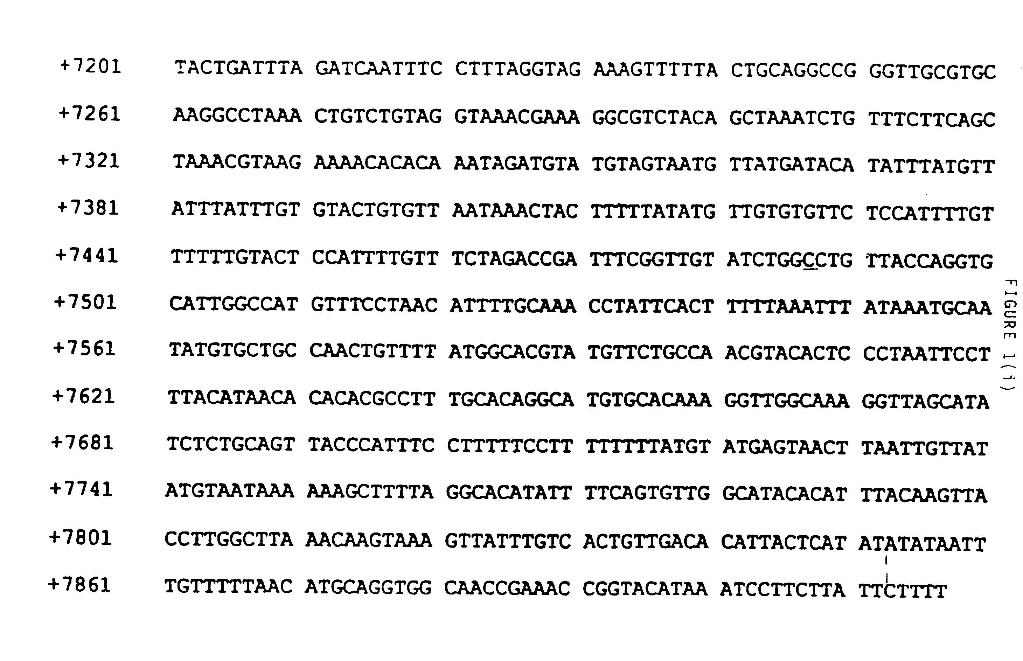

**Fig.1 : 1(a), 1(b), 1(c), 1(d), 1(e), 1(f), 1(g), 1(h), 1(i) :** Séquence nucléotidique du brin d'ADN d'HPV42 analogue à l'ARNm. La position 1 sur le génome circulaire a été déterminée par alignement avec les positions 1 de HPV11 et HPV39.
**Premiers nucléotides :** +1, +1801, +3601 et +7201.

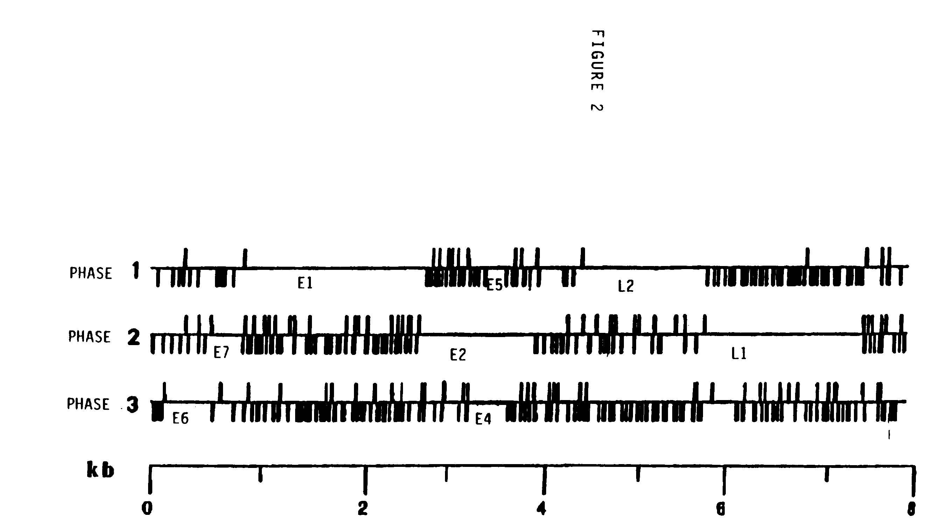

**Fig.2 :** Distribution des codons d'initiation (start) (barres au dessus) et des codons stop (barres au dessous) dans les trois fragments de lecture de l'ADN d'HPV42, de l'ARNm. Les ORFs ont été identifiés par comparaison avec d'autres types d'HPV. La numérotation est en accord avec celle de la Figure 1.

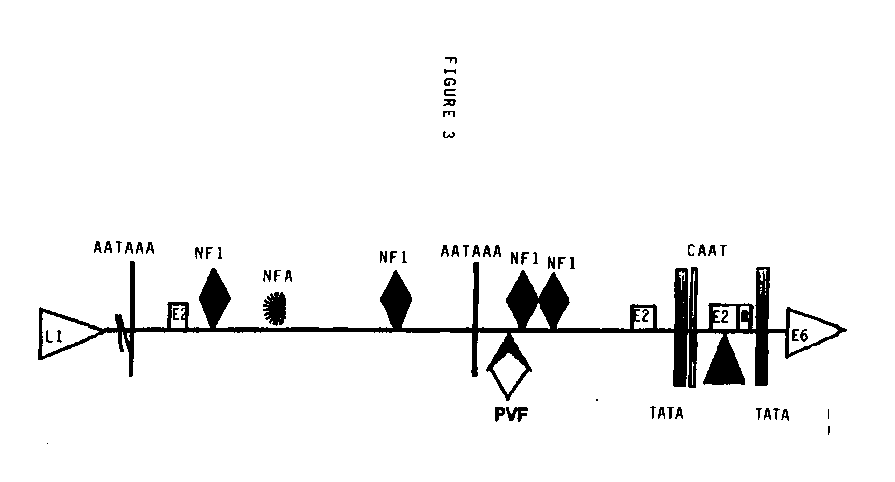

**Fig.3 :** Caractéristiques principales de la région non-codante (NCR). Les motifs de séquences suivants de la NCR s'étendant du nucléotide 7346 jusqu'au nucléotide 113 (nt.7346-113) sont représentés. On spécifie, ci-après, certains des éléments, notamment palindromes de 12 bp spécifiques du papillomavirus (nt.7466, 7883, 44, 59) ; site de polyadénylation : nt.7401 ; boite TATA : nt.6,74 ; boite CAAT : nt.15 ; élément promoteur conservé (AAAGGGAGTA) : nt.34 ; site de liaison au facteur nucléaire 1 (NF1) nt.7503, 7663, 7778, 7803 ; site de liaison du facteur associé (NFA) au facteur (NF1) : nt.7552 ; site de liaison du facteur associé à l'amplificateur de papillomavirus (PVF) (nt.7760).

Le génome de HPV42, propagé dans le plasmide pSP64 a été sous-clôné par fragmentation au hasard (en anglais : "shotgun") dans le phage M13 et séquence par la méthode de terminaison de chaîne didésoxy (14). Des parties correspondantes à 93 % de la totalité du génome ont été obtenues à partir de chacun des brins et chacun des nucléotides a fait l'objet de 5 séquençages. Les régions pour lesquelles subsistait un doute ont été séquencées à nouveau dans les deux directions par la mise en oeuvre dans les deux directions d'oligonucléotides synthétiques utilisés en tant qu'amorces. La séquence longue de 7917 bp (Figure 1) a une teneur en GC de 39,5 %. Le nucléotide +1 a été défini par alignement de la séquence avec celles des HPV de types 11 et 39. La carte de restriction déjà publiée (13) a été confirmée, à l'exception de deux sites supplémentaires Accl aux positions 895 et 943, de part et d'autre d'un site BglII.

L'analyse des séqences correspondant aux phases de lecture ouverte (ORF) a révélée la conservation de l'organisation que l'on retrouve dans tous les génomes de HPV (Figure 2) (15, 39). Tous les ORFs sont localisés sur le même brin d'ADN. La région E4 est incluse de façon complète dans la région E2, tandis que El ainsi que les régions L1 et L2 se superposent partiellement. Ni E4, ni E5 ne possèdent un codon départ ATG (Figure 1 ; Tableau 1). Six signaux de polyadénylation (AATAAA) ont été localisés dans le génome. Deux d'entre eux (nt.4378,7401) consistant en une série de thymidines et purines attenantes et un dinucléotide CA (16, 17) étant placé en aval des groupes de gènes fonctionnellement interconnectés (en anglais "clusters") respectivement précoces et tardifs. Tous sont probablement utilisés pour la polyadénylation. Une région non codante (NCR) d'environ 680 bp est localisée entre le codon d'arrêt de L1 et le codon d'initiation de E6.

Les caractéristiques générales des NCR de tous les HPV sont bien conservées dans HPV42 (Figure 3). Quatre copies du motif d'amplification ACCGNNNNCGGT restent placés sous la dépendance de E2 (nt.444,59,7466,7883) (18,19), des sites de liaison pour les facteurs de transcription de NFl (nt.7503, 7663, 7778, 7803) (21, 22), NFA (nt.7552) (20), une protéine d'amplification de papillomavirus (NFA ; nt.7760) (20), deux boites TATA (nt.6, 74), une boite putative CAAT (nt.15) ainsi qu'un élément de promoteur conservé AAAGGGAGTA (nt.34) (23) sont situés dans le NCR. Aucun élément consensus réagissant de façon évidente à un glucocorticoïde (GRE) n'a été localisé dans le NCR (18, 19, 24). On remarque la présence comme chez HPV39 d'un GRE comportant 3 défauts d'appariement ("mismatches") dans l'ORF de L1 (nt.6555) (8) chez HPV39.

Les protéines E6 et E7 des HPV associées avec des tumeurs malignes ont été reconnues comme jouant un rôle crucial dans la transformation des cellules (25-28). Les motifs Cys-X-X-Cys qui présentent la forme de "doigts de zinc" ("zinc-finger") (29, 30) dont le rôle a été jugé essentiel pour la transformation dans le cas de HPV16, (31) sont présents 4 fois et 2 fois respectivement dans E6 et E7. Des sites potentiels donneurs d'épissage (nt.237), et des sites accepteurs (nt.412) dans l'ORF de E6 et susceptibles de transcription en un ARNm codant pour une protéine épissée E7, E6*, sont bien conservées dans HPV42 (32-34). Jusqu'à ce jour un site d'épissage n'avait été identifié que dans les HPV associés avec des cancers ano-génitaux (6). En outre le motif de division cellulaire de la protéine E7 que l'on avait associée à sa capacité de transformation (31, 35-37) est complètement conservé dans HPV42.

Aucune homologie de séquence prononcée avec d'autres types de HPV, dont les séquences sont connues, n'a pu être mise en évidence. Dans une analyse des homologies assistée par ordinateur, des homologies de l'ordre. de 52 à 56 % ont été obtenues avec les HPV de type 6b, 11, 16, 18, 31 et 33. Il s'agit de valeurs dont les faiblesses sont prononcées lorsqu'on les compare aux homologies de séquences que l'on peut observer entre virus d'un même sous-groupe (4-11). En outre l'HPV42 que l'on associe avec des lésions génitales bénignes, présente des caractéristiques que l'on ne connaissait à ce jour que pour des HPV associés aux seuls carcinomes ; il en est de même en ce qui concerne la présence d'un site E6∗ et du motif de division cellulaire conservée dans la protéine E7. En outre un GRE présent dans les NCRs de tous les HPVs génitaux séquences jusqu'& ce jour (8), est en revanche absent dans le NCR de HPV42. HPV42 peut donc être considéré comme le premier représentant séquencé d'un nouveau groupe de papillomavirus humains.

Il résulte donc de ce qui précède que l'organisation du génome est conservée pour HPV42 comme pour tous les HPVs. Malgré son association à des lésions génitales bénignes, HPV42 présente des caractéristiques qui ont à ce jour été trouvées seulement soit chez des HPVs associés à des carcinomes invasifs, soit dans des HPV non génitaux. Au surplus on ne remarque aucune homologie de séquence étendue de HPV42 avec les HPV connus. La séquence nucléotidique fournie par l'invention témoigne donc de l'identification de la séquence du premier type d'un nouveau sous-groupe de papillomavirus humains.

D'une façon générale, l'invention concerne donc également tout ADN recombinant contenant l'ADN-HPV susdit ou des fragments de cet ADN-HPV, en particulier des sondes d'hybridation formées de ces ADNs recombinants et spécialement adaptées à la détection d'une infection par HPV42 ou d'un variant ou sous-type de ce papillomavirus. Ces sondes peuvent, soit être marquées elles-mêmes, soit être modifiées au niveau de certains nucléotides, notamment en vue de leur couplage, direct ou indirect, avec un marqueur distinct. Il va de soi que dans ces sondes les parties étrangères à la séquence nucléotidique correspondante à l'ADN du papillomavirus et provenant normalement d'un vecteur de clonage, sont telles qu'elles ne risquent pas d'hybrider dans des conditions strictes ou stringentes avec les autres acides nucléiques éventuellement contenus dans l'échantillon testé pour sa teneur éventuelle en ADN du papillomavirus correspondant ou de l'un de ses variants.

Le procédé selon l'invention pour le diagnostic in vitro sur un échantillon biologique à tester, provenant normalement d'un patient humain, d'une infection par un papillomavirus pouvant entraîner ou ayant entraîné une néoplasie génitale, notamment un cancer cervical, vulvaire ou pénien, est donc caractérisé par la mise en contact d'une sonde telle que ci-dessus définie, avec les acides nucléiques de cet échantillon, le cas échéant préalablement rendus accessibles à la sonde, de préférence dans des conditions d'hybridation stringentes, et par la détection de l'hybride formé entre l'ADN viral recherché, éventuellement présent dans l'échantillon et ladite sonde.

Chacune des sondes selon l'invention ou les mélanges contenant la susdite sonde peuvent notamment être utilisés comme suit, étant naturellement entendu que les essais de diagnostic décrits ne sauraient être considérés comme limitatifs des conditions d'emploi dans lesquelles ces sondes ou mélanges de sondes peuvent en fait être utilisés.

Dans l'exemple considéré, il s'agit par exemple d'identifier un HPV de même type que HPV42 dans une biopsie, dans des cellules obtenues par grattage de lésions, ou dans des coupes de biopsies fixées par le mélange de Carnoy (éthanol, chloroforme, acide acétique 6:3:1) et incluses dans la paraffine. L'Examen nécessite l'extraction préalable de l'ADN des prélèvements selon des méthodes dont le principe est connu et met en jeu l'analyse de cet ADN par des expériences d'hybridation moléculaire, effectuées dans du conditions strictes ou moins strictes, à l'aide de sondes radioactives (marquées au ³² p ou au ³⁵ S) préparées à partir de l'HPV selon l'invention ou de mélanges d'ADNs ou d'HPVs le contenant.

Plusieurs méthodes d'hybridation peuvent être utilisées. On peut, par exemple, mettre en oeuvre la méthode d'hybridation sur tache. Cette méthode comporte, après dénaturation de l'ADN, le dépôt d'une quantité aliquote d'ADN sur des membranes (nitrocellulose ou "Genescreenplus", l'hybridation de chaque membrane, dans les conditions usuelles, avec un mélange de sondes et la détection des hybrides radioactifs, par exposition des membranes au contact d'un film radiographique. On peut aussi utiliser une méthode d'hybridation sur réplique. Cette méthode comprend la séparation électrophorétique en gel d'agarose des fragments d'ADN engendrés après traitement de l'ADN par des enzymes de restriction, le transfert des fragments, après dénaturation alcaline, sur des membranes (nitrocellulose, "Genescreenplus") et leur hybridation, dans les conditions usuelles, avec le mélange approprié de sondes. La formation d'hybrides radioactifs est détectée après exposition des membranes au contact d'un film radiographique.

Les sondes radioactives sont constituées soit par des ADNs d'HPVs marqués par la méthode de translation de coupure ("nick-translation"), soit par des ARNs préparés par transcription d'ADNs viraux insérés dans un vecteur, par exemple de type SP6. L'utilisation de sondes radioactives présente l'avantage d'une grande sensibilité, mais ceci n'exclut pas l'utilisation de sondes non radioactives, par exemple de sondes biotinylées et susceptibles d'être reconnues par des anticorps soit marqués eux-mêmes, soit eux-mêmes reconnus par des anticorps portant un marqueur enzymatique, fluorescent, etc...

L'invention concerne également des cultures cellulaires compétentes transformées avec des ADNs recombinants du type sus-indiqué, en particulier ceux dans lesquels la séquence nucléotidique correspondant à l'ADN ou la séquence de l'ADN de HPV39 est placée sous le contrôle d'éléments de transcription et/ou de régulation de cette séquence nucléotidique dans ladite culture cellulaire.

En outre et compte tenu des caractéristiques nouvellement découvertes de HPV42, son utilisation pour confirmer - ou infirmer - un diagnostic in vitro de carcinome potentiel ou déjà existant découlant de l'utilisation - notamment dans les conditions rappelées plus haut - de sondes mettant en jeu des HPVs présentant des caractéristiques qui se rapprochent de celles de HPV42, en particulier au niveau de leurs ORFs de E6, E6∗ et/ou E7. Il s'agit plus particulièrement d'un ou de plusieurs des HPVs suivants : HPV6, HPV11, HPV16, HPV18, HW31, HPV33, HPV39 et HPV57.

L'invention concerne donc plus particulièrement des kits de diagnostics in vitro comprenant :
- d'une part, une sonde contenant un ADN correspondant à tout ou partie de l'ADN de HPV42 ou d'un ADN hybridant avec le précédent dans des conditions strictes ;
- d'autre part, d'au moins une sonde contenant un ADN correspondant à tout ou partie de l'ADN de l'un au moins des papillomavirus HPV6, HPV11, HW16, HPV18, HPV31, HPV33, HPV39 et HPV57.

Il apparaîtra clairement au spécialiste que la réalisation d'essais d'hybridation effectués dans les conditions qui seront encore indiquées plus loin (en particulier dans des conditions d'hybridation stricte) avec ces ensembles de sondes, permettra au spécialiste soit de confirmer, soit à tout le moins de nuancer un diagnostic de carcinome potentiel ou existant, soit même d'infirmer un tel diagnostic. En particulier le diagnostic de carcinome potentiel ou existant pourrait être confirmé dans l'hypothèse où l'on constaterait une absence d'hybridation de l'ADN de papillomavirus contenu dans le prélèvement le contenant avec l'ADN de HPV42, mais une réponse positive dans les essais d'hybridations avec l'un ou les autres ADNs du kit. En revanche une hybridation positive également avec l'ADN de HPV42 permettrait, selon le cas, soit de nuancer, soit d'infirmer un diagnostic pessimiste de ce type. Pour obtenir un diagnostic plus net, il peut être nécessaire de compléter les essais d'hybridation avec des sondes contenant des fragments plus spécifiques issus de l'ADN de HPV42, plus particulièrement de fragments comprenant tout ou partie des fragments qui, par exemple, s'étendent entre les nucléotides d'extrémités respectivement indiqués ci-après : nt 7342 à nt 870.

Parmi ces fragments, on donnera souvent la préférence à ceux qui contiennent plus particulièrement tout ou partie des ORFs E6* ou E7 ou encore tout ou partie du NCR dépourvu de site GRE et qui s'étend entre nt 7342 et nt 108, notamment de nt 7556 à nt 7576.

L'invention concerne encore des séquences d'au moins 15 nucléotides issus ou dérivés de la séquence totale de HPV42, voire de 20 à 40 nucléotides utilisables à titre d'amorces de PCR et permettant des analyses fines d'identification d'un ADN de papillomavirus par comparaison avec des séquences nucléotidiques caractéristiques de papillomavirus. Des amorces préférées sont issues des séquences nucléotidiques qui ont été identifiées plus haut. Ces amorces se présentent sous forme bicaténaire ou monocaténaire.

En rapport avec ce qui précède, l'invention concerne donc un procédé pour détecter in vitro, la présence soit d'un acide nucléique codant pour HPV42, soit d'un ARNm, comprenant notamment les étapes suivantes :
**a)** la mise en contact, en présence de nucléosides triphosphates et d'un agent inducteur de polymérisation (polymerase ou reverse transcriptase selon le cas), de l'échantillon biologique suspecté de contenir un ADN apparenté à HPV42, préalablement rendu accessible à une première amorce dans des conditions autorisant l'hybridation entre ces amorces et l'ADN, recherché, et la polymérisation à partir de ces amorces hybridées à l'ARNm ou l'ADNc, pour produire un duplex formé entre le produit d'allongement de l'amorce, hybridé soit avec le brin d'acide nucléique recherché, soit avec l'ARNm correspondant lorsqu'ils sont présents dans l'échantillon biologique,
**b)** la dénaturation du duplex obtenu à l'étape a) de façon à "séparer" le produit d'allongement de l'amorce de l'ADN ou de l'ARNm à détecter,
**c)** la mise en contact du produit d'allongement obtenu avec une deuxième amorce (au sens que l'on adonné plus haut de cette expression) ayant une séquence de nucléotides (1) non complémentaire de celle de la première amorce et (2) complémentaire d'une séquence du produit d'allongement précédemment formé,
**d)** le cas échéant la répétition des étapes a) et b) et c) en présence des première et deuxième amorces utilisées en excès et des réactifs nécessaires à la production de nouveaux produits d'allongement utilisés leur tour comme matrice pour des synthèses supplémentaires, jusqu'à obtenir une quantité suffisante pour être détectée de produits d'allongement des amorces utilisées.
**e)** la détection de la présence des produits d'allongement caractéristiques de la présence de l'ADN de HPV42 ou de l'ARN correspondant.

La mise en oeuvre du procédé de détection ci-dessus est réalisée à partir d'un échantillon biologique prélevé chez un patient et constitué par exemple par une biopsie, une pièce opératoire, un fluide biologique.

Pour plus de détails concernant la technique relative au procédé de détection décrit ci-dessus, ou pour l'élaboration de variantes de ce procédé, l'homme du métier pourra se reporter utilement aux principes décrits dans les brevets US.4.683.202 et US.4.683.195.

Quel que soit le protocole utilisé de PCR ou, plus généralement, de méthode d'analyse reposant sur une amplification de séquences génétiques issues de HPV42, l'on comprendra que les chaînes de polymérisation seront d'autant plus longues que l'identité de l'ADN de papillomavirus détecté, du moins au niveau de la région concernée, sera plus proche de la région de HPV42 dont sont issues les sondes.

Les tableaux qui suivent fournissent encore des éléments analytiques relatifs :
- au caractéristiques principales du génome de HPV42,
- à des résultats d'analyse comparative (pourcentages d'homologie de séquences entre HPV42 et les génomes d'autres papillomavirus).

Enfin la bibliographie des publications auxquelles il est fait référence dans le présent texte est fournie à la fin de cette description.

**TABLEAU 1**

| PRINCIPAUX CARACTERES DU GENOME DE HPV 42 | | | | | |
|---|---|---|---|---|---|
| ORF | PREMIER NUCLEOTIDE | PREMIER ATG | NUCLEOTIDE PRECEDANT LE CODON STOP | ORF TAILLE (bp) | POIDS MOLECULAIRE PREVISIBLE (kD) |
| E6 | 108 | 114 | 563 | 450 | 17.5 |
| E7 | 476 | 542 | 820 | 279 | 10.7 |
| E1 | 724 | 829 | 2757 | 1929 | 72 |
| E2 | 2672 | 2702 | 3895 | 1194 | 45.2 |
| E4 | 3282 | ----- | 3641 | 360 | 13.4 |
| E5 | 3919 | ----- | 4203 | 285 | 10.6 |
| L2 | 4348 | 4423 | 5853 | 1431 | 51.2 |
| L1 | 5756 | 5837 | 7342 | 1506 | 56.1 |
| : A PARTIR DU PREMIER ATG, A L'EXCEPTION DE E4 ET E5 | | | | | |

**TABLEAU 2**

| ANALYSE COMPARATIVE DES GENOMES DE HPVs | | | | | | | | |
|---|---|---|---|---|---|---|---|---|
| | HPV1 | HPV6 | HPV8 | HPV11 | HPV16 | HPV18 | HPV31 | HPV33 |
| DNA^{a} | n.t | 56 | n.t | 54 | 55 | 52 | 52 | 53 |
| E6^{b} | 30.7 | 48.4 | 27.1 | 49.0 | 41.3 | 39.4 | 40.6 | 41.3 |
| E7^{b} | 44.1 | 60.3 | 35.5 | 59.6 | 55.9 | 49.5 | 61.3 | 60.2 |
| E1^{b} | 43.1 | 63.8 | 42.8 | 60.7 | 44.3 | 57.7 | 53.7 | 55.2 |
| E2^{b} | 32.5 | 46.3 | 36.0 | 50.0 | 35.5 | 42.0 | 30.3 | 43.8 |
| E4^{b} | 31.8 | 32.6 | 17.0 | 32.0 | 36.2 | 33.3 | 31.7 | 37.9 |
| E5^{b} | n.t. | 20.7 | n.t. | 24.8 | 34.3 | 30.7 | 35.1 | 28.3 |
| L2^{b} | 42.1 | 56.4 | 46.3 | 56.8 | 59.7 | 47.2 | 48.0 | 58.3 |
| L1^{b} | 53.2 | 69,5 | 49.6 | 68.9 | 73.3 | 63.7 | 74.3 | 71.3 |

| | | | | | | | | |
|---|---|---|---|---|---|---|---|---|
| LES VALEURS REPRESENTENT DES POURCENTAGES D'HOMOLOGIE APRES ALIGNEMENT AVEC LE PROGRAMME DE NEEDLEMAN ET WUNSCH (38) n.t.: NON TESTE a: % D'HOMOLOGIE DE NUCLEOTIDES | | | | | | | | |
| b: % HOMOLOGIE EN AMINOACIDES | | | | | | | | |

### REFERENCES

1 de Villiers, E, J. Virol. **63,** 4898-4903 (1989).
2. Schwarz E., Dürst, M., Demankowski, C., Lattermann. O., Zech, R., Wolfsperger, E, Suhai, S. and zur Hansen, H., EMBO J. **2**, 2341.2348 (1983).
3. Dartmann, K., Schwarz, E., Gissmann, L. and zur Hausen, H., Virology **151**, 124-130 (1986).
4. Seedorf, K., Krämer, G., Dürst, M., Suhai, S. nd Röwekamp, W.G., Virology **145,** 181-185 (1985).
5. Cole, S.T. and Danos, O.J., J. Mol. Biol. **193**, 599-608 (1987).
6. Goldsborough, M.D., DiSilvestre, D., Temple, G.F. and Lorincz, A.T., Virology **171**, 306-311 (1989).
7. Cole, S.T. and Streeck, R.E., J. Virol **56,** 85-91 (1986).
8. Volpers, C. and Streeck, R.E, Virology **181,** 419-423 (1991).
9. Zachow, K.R., Ostrow, R.S. and Faras, A.J., Virology **158,** 251-254 (1987).
10. Fuchs, P.G., Iftner, T., Weninger, J., and Pfister, H., J. Virol**. 58,** 626-634 (1986).
11. Kiyono, T., Adachi, A. and Ishibashi, M., Virology **177,** 401-405 (1990).
12. Beaudenon, S., Praetorius, F., Kremsdorf, D., Lutzner, M., Worsaae, N., Pehan-Arnaudet, G. and Orth, G., J. Invest. Dermatol. **88,** 130-135 (1987).
13. Beaudenon, S., Kremsdorf, D., Obalek, S., Jablonska, S., Croissant, O., Pehan-Arnaudet, G. and Orth, G., Virology **161,** 374-384 (1987).
14. Sanger, F., Nicklen, S. and Coulson, A.R., Proc. Natl. Acad. Sci. USA **74,** 5463-5467 (1977).
15. Knippers, R. in Curr. Top. Microbiol. Immunol. 144 (Knippers, R. and Levine, A.J., Eds.), 137-142 (1989).
16. Birnstiel, M.L., Busslinger, M. and Strab, K. Cell **41,** 349-359 (1985).
17. Weiss, E.A., Gilmartn, G.M. and Nevins, J.R., EMBO J. **10,** 215-219 (1991).
18. Gloss, B., Bernard, H.U., Seedort, K. and Klock, G., EMBO J. **12,** 735-3743 (1987).
19. Garcia-Garranca, A., Thierry, F. and Yaniv, M., J. Virol, **62,** 4321-4330 (1988).
20. Chong, T., Chan, W.K. and Bernard, H.U., Nucl. Acids Res. **18,** 465-470 (1990).
21. Benoist, C. and Chambon, P., Nature **290,** 304-310 (1981).
22. Wingender, E., Nucl. Acids Res. **16,** 1879-1902 (1988).
23. Gloss, B., Chong, T, and Beranrd, H.U., J. Virol. **63,** 1142-1152 (1989).
24. Jantzen, H.M., Strähle, U., Gloss, B., Stewart, F., Schmid, W., Boshart, M., Miksicek, R. and Schütz, G., Cell **49**, 29-38 (1987).
25. Lamberti, C., Morrissey, L.C., Grossman, S.T. and Androphy, E.J., EMBO J. **9**, 1907-1913 (1990).
26. Pecoraro, G., Morgan, D. and Defendi, V., Proc. Natl. Acad. Sci. USA **86**, 563-567 (1989).
27. Halbert, C., Demers, G.W. and Galloway, D.A., J. Virol. **65,** 473-478 (1991).
28. Storey, A., Pim, D., Murray, A., Osborn, K., Banks, L. and Crawford, L., EMBO J. 7, 1815-1820 (1988).
29. Miller, J., McLachlan, A.D. and Klug, A., EMBO J. **4**, 1609-1614 (1985).
30. Berg, J.M., Science **232,** 485-487 (1986).
31. Storey, A., Almond, N., Osborn, K. and Crawford, L., J. Gen. Virol. **71,** 965-970 (1990).
32. Schneider-Gädiske, A, and Schwarz, E., EMBO J. **5,** 2285-2292 (1986).
33. Seedorf, K., Oltersdorf, T., Krämmer, G. and Röwekamp, W., EMBO J. **6**, 139-144 (1987).
34. Smotkin, D. and Wettstein, F.O., Proc. Natl. Acad. Sci. USA **83,** 4680-4684 (1986).
35. Phelps, W.C., Yee, C.L., Münger, K. and Howley, P.M., Cell **53.** 539-547 (1988).
36. Münger, K., Weness, B.A., Dyson, N., Phelps, W.C., Harlow, E. and Howley, P.M., EMBO J. **8**, 4099-4105 (1989).
37. Figge, J. and Smith, T.F., Nature **334,** 109 (1988).
38. Needleman, S.B. and Wunsch, C.D., J. Mol. Biol. **48,** 443-450 (1970).
39. Koutsky, L.A., Galloway, D.A. and Holmes, K.K., Am. J. Epidemiol. Rev. **10,** 122-163 (1988).

## Revendications

1. ADN issu d'un papillomavirus HPV42 caractérisé en ce qu'il consiste essentiellement en l'ADN caractérisé par la séquence de nucléotides de la figure 1 ou d'un fragment d'au moins 15 nucléotides de celui-ci, ou encore en un ADN d'au moins 15 nucléotides ou fragment d'ADN correspondant d'au moins 15 nucléotides hybridant avec les précédents dans des conditions strictes.

2. ADN selon la revendication 1, caractérisé en ce que sa séquence correspond à l'une de celles définies ci-après par les positions ci-après indiquées de leurs nucléotides d'extrémités respectives nt 7342 à nt 870 ou à tout fragment d'au moins 15 nucléotides contenu dans cette séquence ou encore à tout fragment d'ADN d'au moins 15 nucléotides hybridant avec les précédents dans des conditions strictes.

3. ADN selon la revendication 1, caractérisé en ce que la séquence contient l'une des séquences suivantes : nt 7342 à nt 108, notamment de nt 7556 à 7576.

4. ADN selon la revendication 1 ou 2, caractérisé en ce que ledit fragment correspond à l'un des gènes suivants de HPV42 : E1, E2, E4, L1, L2 ou à tout ou partie de la région intergénique NC.

5. ADN selon la revendication 1 ou 2, caractérisé en ce que ledit fragment correspondant à l'un des gènes suivants de HPV42 : E6 ou E7.

6. ADN selon la revendication 1 ou 2, caractérisé en ce que sa séquence correspond à celle d'une région non codante (NCR) s'étendant entre les nucléotides 7342 et 108 ou à tout fragment d'au moins 15 nucléotides contenu dans cette séquence et en ce que ce NCR ou fragment est dépourvu d'élément consensus réagissant à un glucocorticoïde (GRE).

7. Sonde d'hybridation constituée par un ADN dérivé de l'ADN selon l'une quelconque des revendications 1 à 6.

8. Amorce de PCR comportant au moins 15, de préférence 20 à 40 nucléotides, cette amorce étant issue de l'un des ADN ou fragment d'ADN selon l'une quelconque des revendications 1 à 6.

9. Procédé de détection in vitro sur un échantillon biologique à tester, d'une infection par un papillomavirus du type HPV42, caractérisé par la mise en contact d'une sonde correspondante, telle que définie dans la revendication 6, avec les acides nucléiques de cet échantillon, le cas échéant préalablement rendus accessibles à la sonde, de préférence dans des conditions d'hybridation stringente, et par la détection de l'hybride formé entre l'ADN viral recherché, éventuellement présent dans l'échantillon, et ladite sonde.

10. Procédé selon la revendication 9, caractérisé en ce que le diagnostic in vitro est orienté vers la détection de condylomas ou de papillomas plats non susceptibles de transformations en carcinomes.

11. Kit de diagnostic permettant la confirmation ou l'infirmation d'un diagnostic in vitro du caractère carcinomique potentiel ou effectif d'une infection au papillomavirus comportant :
- d'une part, une sonde contenant un ADN correspondant à tout ou partie de l'ADN de HPV42 ou d'un ADN hybridant avec le précédent dans des conditions strictes ;
- d'autre part, au moins une sonde contenant un ADN correspondant à tout ou partie de l'ADN de l'un au moins des papillomavirus HPV6, HPV11, HPV16, HPV18, HPV31, HPV33, HPV39 et HPV57.

12. Application des deux amorces distinctes conformes à la revendication 8 à la détection et l'analyse fine d'un ADN de papillomavirus contenu dans un échantillon biologique en égard à son identité à celui de HPV42, au moins au niveau des régions correspondantes de leurs ADNs génomiques respectifs, ou à leurs degrés d'analogie respectifs.

## Claims

1. DNA derived from a HPV42 papillomavirus characterized in that it consists essentially of the DNA characterized by the nucleotide sequence of Figure 1 or by a fragment of at least 15 nucleotides of this latter, or also of a corresponding DNA of at least 15 nucleotides or DNA fragment of at least 15 nucleotides which hybridizes with the former under stringent conditions.

2. DNA according to Claim 1, characterized in that its sequence corresponds to one of those defined below by the positions indicated below for their end nucleotides nt.7342 and nt.870, respectively, or to any fragment of at least 15 nucleotides contained in this sequence or also to any DNA fragment of at least 15 nucleotides which hybridizes with the former under stringent conditions.

3. DNA according to Claim 1, characterized in that the sequence contains one of the following sequences: nt.7342 to nt.108, in particular from nt.7556 to 7576.

4. DNA according to Claim 1 or 2, characterized in that the said fragment corresponds to one of the following HPV42 genes : E1, E2, E4, L1, L2 or to all or part of the intergenic region NC.

5. DNA according to Claim 1 or 2, characterized in that the said fragment corresponds to one of the following genes of HPV42 : E6 or E7.

6. DNA according to Claim 1 or 2, characterized in that its sequence corresponds to that of a non-coding region (NCR) extending from nucleotides 7342 to nucleotide 108 or to any fragment of at least 15 nucleotides contained in this sequence and in that this NCR or fragment lacks a consensus element which interacts with a glucocorticoid (GRE).

7. Hybridization probe constituted by a DNA derived from the DNA according to any one of the Claims 1 to 6.

8. Primer for PCR containing at least 15 and preferably 20 to 40 nucleotides, this primer being derived from one of the DNAs or DNA fragments according to any one of the Claims 1 to 6.

9. In vitro detection procedure for an infection by a HPV42-type papillomavirus in a biological test sample, characterized by the placing of a corresponding probe, such as defined in Claim 6, in contact with the nucleic acids of this sample, where necessary made accessible to the probe beforehand, preferably under stringent hybridization conditions, and by detection of the hybrid formed between the viral DNA under investigation which might be present in the sample and the said probe.

10. Procedure according to Claim 9, characterized in that the in vitro diagnosis is oriented towards the detection of condylomas or flat papillomas not susceptible of being transformed into carcinomas.

11. Diagnostic kit making it possible to confirm or refute an in vitro diagnosis of the actual or potential carcinogenic character of an infection with a papillomavirus, containing:
- on the one hand, a probe containing a DNA corresponding to all or part of the HPV42 DNA or of a DNA which hybridizes with the former under stringent conditions;
- on the other hand, at least a probe containing a DNA corresponding to all or part of the DNA of at least one of the papillomaviruses HPV6, HPV11, HPV16, HPV18, HPV31, HPV33, HPV39 and HPV57.

12. Use of the two distinct primers in conformity with Claim 8 for the detection and precise analysis of a papillomavirus DNA contained in a biological sample in regard of its identity with that of HPV42, at least in corresponding regions of their respective genomic DNAs, or to their respective degrees of homology.

## Patentansprüche

1. Von einem Papillomavirus HPV42 stammende DNA, dadurch gekennzeichnet, daß sie im wesentlichen aus DNA, die durch die Nukleotidsequenz der Figur 1 oder eines Fragments von jener mit mindestens 15 Nukleotiden gekennzeichnet ist, oder ferner aus einer entsprechenden DNA mit mindestens 15 Nukleotiden oder einem entsprechenden DNA-Fragment mit mindestens 15 Nukleotiden, die mit den Vorangehenden unter stringenten Bedingungen hybridisieren, besteht.

2. DNA nach Anspruch 1,
dadurch gekennzeichnet, daß deren Sequenz einer von jenen, die nachfolgend durch die nachfolgend für deren jeweilige Endnukleotide angegebenen Positionen, Nukleotid 7342 bis Nukleotid 870, definiert sind, oder jedem beliebigen Fragment mit mindestens 15 Nukleotiden, das in dieser Sequenz enthalten ist, oder ferner jedem beliebigen DNA-Fragment mit mindestens 15 Nukleotiden, das mit den Vorangehenden unter stringenten Bedingungen hybridisiert, entspricht.

3. DNA nach Anspruch 1,
dadurch gekennzeichnet, daß die Sequenz eine der folgenden Sequenzen enthält: Nukleotid 7342 bis Nukleotid 108, insbesondere von Nukleotid 7556 bis 7576.

4. DNA nach Anspruch 1 oder 2,
dadurch gekennzeichnet, daß das Fragment einem der folgenden Gene von HPV42 entspricht: E1, E2, E4, L1, L2 oder der Gesamtheit oder einem Teil des intergenischen nicht-kodierenden (NC-) Bereichs.

5. DNA nach Anspruch 1 oder 2,
dadurch gekennzeichnet, daß das Fragment einem der folgenden Gene von HPV42 entspricht: E6 oder E7.

6. DNA nach Anspruch 1 oder 2,
dadurch gekennzeichnet, daß deren Sequenz jener eines nicht-kodierenden Bereichs (NCR), der sich zwischen den Nukleotiden 7342 und 108 erstreckt, oder jedem beliebigen Fragment mit mindestens 15 Nukleotiden, das in dieser Sequenz enthalten ist, entspricht und daß dieser NCR oder dieses Fragment keine Glucocorticoid-responsiven Consensuselemente ("consensus glucocorticoid responsive elements"; GRE) enthält.

7. Hybridisierungssonde, gebildet durch eine von der DNA nach einem der Ansprüche 1 bis 6 abgeleitete DNA.

8. PCR-Primer, umfassend mindestens 15, vorzugsweise 20 bis 40 Nukleotide, wobei dieser Primer von einer der DNAs oder einem DNA-Fragment nach einem der Ansprüche 1 bis 6 abstammt.

9. in vitro-Nachweisverfahren für eine Infektion durch ein Papillomavirus des Typs HPV42 bei einer zu untersuchenden biologischen Probe,
gekennzeichnet durch das Inkontaktbringen einer entsprechenden Sonde, wie sie in Anspruch 6 definiert ist, mit den Nukleinsäuren dieser Probe, die gegebenenfalls vorab für die Sonde zugänglich gemacht worden sind, vorzugsweise unter stringenten Hybridisierungsbedingungen und durch den Nachweis des Hybridisierungsprodukts, das zwischen der gesuchten viralen DNA, die gegebenenfalls in der Probe vorhanden ist, und der Sonde gebildet worden ist.

10. Verfahren nach Anspruch 9,
dadurch gekennzeichnet, daß die in vitro-Diagnostik auf den Nachweis von Kondylomen oder von flachen Papillomen, die zu Transformationen zu Karzinomen nicht imstande sind, ausgerichtet ist.

11. Diagnosekit, welcher die Bestätigung oder die Entkräftung einer in vitro-Diagnostik des potentiellen oder tatsächlichen karzinomischen Charakters einer Papillomavirus-Infektion ermöglicht,
umfassend:
- einerseits eine Sonde, die eine DNA enthält, die der Gesamtheit oder einem Teil der DNA von HPV42 oder einer DNA, die mit der Vorangehenden unter stringenten Bedingungen hybridisiert, entspricht,
- andererseits mindestens eine Sonde, die eine DNA enthält, die der Gesamtheit oder einem Teil der DNA von mindestens einem der Papillomaviren HPV6, HPV11, HPV16, HPV18, HPV31, HPV33, HPV39 und HPV57 entspricht.

12. Anwendung von zwei verschiedenen Primern gemäß Anspruch 8 für den Nachweis und die Feinanalyse einer Papillomavirus-DNA, die in einer biologischen Probe enthalten ist, hinsichtlich ihrer Identität mit jener von HPV42, zumindest auf Ebene der entsprechenden Bereiche von deren jeweiligen genomischen DNAs oder ihrer jeweiligen Verwandtschaftsgrade.
